# EUROPEAN PATENT SPECIFICATION

(11) **EP 3 810 206 B1**
(45) Date of publication and mention of the grant of the patent: **24.01.2024**
(21) Application number: 19732641.6
(22) Date of filing: 21.06.2019
(51) Int. Cl.: A61K 48/00, C12N 15/86, C12N 15/88

(54) **COMBINATORIAL GENE THERAPY**
KOMBINATORISCHE GENTHERAPIE
THÉRAPIE GÉNIQUE COMBINATOIRE

(30) Priority: 22.06.2018 GB 201810301
(43) Date of publication of application: 28.04.2021
(73) Proprietor: Evox Therapeutics Ltd, Oxford OX4 4HG (GB)
(72) Inventor: EL ANDALOUSSI, Samir, 14131 Huddinge (SE); LUNDIN, Per, Oxford OX4 4HG/GB (GB)
(74) Representative: Cooley (UK) LLP
(86) International application number: PCT/EP2019/066471
(87) International publication number: WO 2019/243574

(56) References cited:
- WO-A1-2012/123430
- WO-A1-2016/044947
- WO-A1-2017/172606
- KAI O. BÖKER ET AL: "The Impact of the CD9 Tetraspanin on Lentivirus Infectivity and Exosome Secretion", MOLECULAR THERAPY, vol. 26, no. 2, February 2018 (2018-02), pages 634-647, XP055609924, ISSN: 1525-0016, DOI: 10.1016/j.ymthe.2017.11.008
- ESTHER NOLTE-'T HOEN ET AL: "Extracellular vesicles and viruses: Are they close relatives?", PNAS, vol. 113, no. 33, 18 July 2016 (2016-07-18), pages 9155-9161, XP055609891, US ISSN: 0027-8424, DOI: 10.1073/pnas.1605146113
- HALL JUSTIN ET AL: "Delivery of Therapeutic Proteins via Extracellular Vesicles: Review and Potential Treatments for Parkinson's Disease, Glioma, and Schwannoma", CELLULAR AND MOLECULAR NEUROBIOLOGY, SPRINGER NEW YORK, US, vol. 36, no. 3, 26 March 2016 (2016-03-26) , pages 417-427, XP035904161, ISSN: 0272-4340, DOI: 10.1007/S10571-015-0309-0 [retrieved on 2016-03-26]
- DHRUVITKUMAR S. SUTARIA ET AL: "Achieving the Promise of Therapeutic Extracellular Vesicles: The Devil is in Details of Therapeutic Loading", PHARMACEUTICAL RESEARCH, vol. 34, no. 5, 17 March 2017 (2017-03-17) , pages 1053-1066, XP55543423, US ISSN: 0724-8741, DOI: 10.1007/s11095-017-2123-5
- LARA TIMANTRA SCHILLER ET AL: "Enhanced Production of Exosome-Associated AAV by Overexpression of the Tetraspanin CD9", MOLECULAR THERAPY - METHODS & CLINICAL DEVELOP, vol. 9, 29 March 2018 (2018-03-29), pages 278-287, XP055609975, GB ISSN: 2329-0501, DOI: 10.1016/j.omtm.2018.03.008
- EDIT I. BUZAS ET AL: "Emerging role of extracellular vesicles in inflammatory diseases", NATURE REVIEWS RHEUMATOLOGY, vol. 10, no. 6, 18 February 2014 (2014-02-18), pages 356-364, XP55418387, GB ISSN: 1759-4790, DOI: 10.1038/nrrheum.2014.19
- AMINE MELIANI ET AL: "Enhanced liver gene transfer and evasion of preexisting humoral immunity with exosome-enveloped AAV vectors", BLOOD ADVANCES, vol. 1, no. 23, 24 October 2017 (2017-10-24), pages 2019-2031, XP055576464, ISSN: 2473-9529, DOI: 10.1182/bloodadvances.2017010181
- MENTKOWSKI KYLE I ET AL: "Therapeutic Potential of Engineered Extracellular Vesicles", THE AAPS JOURNAL, SPRINGER INTERNATIONAL PUBLISHING, CHAM, vol. 20, no. 3, 15 March 2018 (2018-03-15) , pages 1-17, XP036517398, DOI: 10.1208/S12248-018-0211-Z [retrieved on 2018-03-15]
- BESSIS N ET AL: "Immune responses to gene therapy vectors: influence on vector function and effector mechanisms", GENE THERAPY, NATURE PUBLISHING GROUP, LONDON, GB, vol. 11, no. Suppl 1, 29 September 2004 (2004-09-29), pages S10-S17, XP037772660, ISSN: 0969-7128, DOI: 10.1038/SJ.GT.3302364 [retrieved on 2004-09-29]
- GOWEN AUSTIN ET AL: "Mesenchymal Stem Cell-Derived Extracellular Vesicles: Challenges in Clinical Applications", FRONTIERS IN CELL AND DEVELOPMENTAL BIOLOGY, vol. 8, 12 March 2020 (2020-03-12), XP055915655, DOI: 10.3389/fcell.2020.00149
- Taeko Shigemoto-Kuroda ET AL: "MSC-derived Extracellular Vesicles Attenuate Immune Responses in Two Autoimmune Murine Models: Type 1 Diabetes and Uveoretinitis", STEM CELL REPORTS, vol. 8, no. 5, 1 May 2017 (2017-05-01), pages 1214-1225, XP055579346, United States ISSN: 2213-6711, DOI: 10.1016/j.stemcr.2017.04.008

## Description

### Technical field

The present invention relates to administration of gene therapy, in particular the development of effective combinatorial strategies for gene delivery in inter alia monogenetic diseases.

### Background art

Gene therapy aims to correct defective genes that underlie the development of diseases. A common approach to addressing this issue involves the delivery of a normal gene to the nucleus. This gene may then be inserted into the genome of the targeted cell or may remain episomal. Delivery of a corrective gene to a subject's target cells can be carried out via numerous methods, including the use of viral vectors. Multiple recombinant gene transfer vectors based on different types of viruses (e.g. retroviruses, adenoviruses, adeno-associated viruses, lentiviruses, etc.) have been developed and tested in clinical trials in recent years. Gene transfer vectors based on adeno-associated virus (AAV) have become favoured vectors because of characteristics such as an ability to transduce different types of dividing and non-dividing cells of different tissues and the ability to establish stable, long-term transgene expression.

There are several disadvantages to the use of all viral vectors for gene delivery: (1) a large number of clinically relevant cell types and tissues are not efficiently transduced with any type of vector, (2) a considerable proportion of patients have pre-existing antibodies (i.e. are seropositive) to many viral vectors due to prior exposure to wildtype virus, resulting in elimination or considerable reduction in virus-mediated transduction, (3) induction of humoral, innate and cellular immune responses have been reported against most if not all viral vectors and even against AAVs (Calcedo et al., Hum Gene Ther Methods, 2018), (4) in diseases where the pathology is caused by a complete or partial lack of a particular protein, the expression of a transgene product may trigger a T cell-mediated immune response against the therapeutic protein itself or against cells transduced with the viral vector (Hollinger & Chamberlain, Curr Opin Neurol, 2018; Sherman et al., Front Immunol, 2017), (5) virus-mediated expression of a particular transgene product is uncontrollable and often difficult to predict, which may lead to gene expression levels that are either too high, thereby leading to toxicity, or too low, thus resulting in a lack of therapeutic activity, and, finally, (6) episomal gene expression (for instance as a result of AAV-mediated gene therapy) is automatically decreasing over time as a result of DNA dilution in growing organs, which is a considerable problem for especially paediatric monogenetic disorders.

Thus, although gene therapy represents a highly tractable approach to the treatment of monogenetic disorders the inability to safely and effectively administer viral vectors more than once is a significant limitation to this technology. Various approaches have been developed to enable repeat administration of viral vectors and to avoid immune responses against the transgene itself, including immunosuppressive treatment protocols (Sherman et al., Front Immunol, 2017; Unzu et al., J Trans Med, 2012), development of chimeric virus serotypes against which patients are not seropositive, and intra-amniotic gene delivery while the immune system is developing (Hayashita-Kinoh et al., Mol Ther, 2015). However, repeat administration of viral gene therapy remains a considerable challenge for any gene therapy application and in particular for genetic null disorders.

### Summary of the invention

The invention is as defined in the claims.

It is hence an object of the present invention to overcome the above-identified problems associated with administration of genetic therapies, in particular the problems of repeat dosing of a transgene polynucleotide and the triggering of immune responses against the transgene and/or the vector itself. Furthermore, the present invention aims to satisfy other existing needs within the art, for instance to develop a safe and effective therapeutic strategy for gene therapy as a viable long-term treatment paradigm even in cases with significant immuno-reactivity against the adeno-associated virus(AAV)-based vector.

The present invention achieves these and other objectives by utilizing an inventive combinatorial approach, taking advantage of the unique strengths of viral gene therapy and exosome-mediated gene delivery. In a first aspect, the present invention relates to a combination therapy comprising essentially two main components, namely (i) a AAV-based vector comprising a transgene encoding for a protein of interest (POI), and (ii) an extracellular vesicle (EV)-based vector comprising the transgene in the form of any DNA, RNA or DNA/RNA polynucleotide, for instance an mRNA or any other type of linear RNA polynucleotide, a circular RNA (circRNA), a mini-circle DNA, a linear DNA polynucleotide, plasmid DNA (pDNA), and/or any type of polynucleotide comprising a both RNA and DNA residues. The POI may be a protein, a peptide, any part of a protein or a peptide, or any combination thereof, or any POI fused to one or more domain(s), region(s), construct(s), site(s) or linker(s) that would enhance its therapeutic activity or potency. Although not part of the invention, the POI can also be delivered in the form of a protein or peptide, i.e. by engineering the EV-based vector to deliver the POI in the form of a protein. Engineering of EVs, such as exosomes, to comprise a protein and/or peptide of interest can be achieved, for instance, by overexpressing the POI in an EV-producing cell or by fusing the POI to an exosomal polypeptide followed by expression of such a genetic construct in an EV-producing, to enable directed EV loading of the POI into the EV-based vector. Thus, in some embodiments, not part of the invention, an AAV-based vector delivering a transgene encoding for a POI may be combined with an EV-based vector delivering the POI in the form of a protein. In a further embodiment, the EV-based vector may comprise both the transgene in the form of a polynucleotide and the POI for which the transgene encodes, and this EV-based vector may advantageously be combined with an AAV-based vector encoding for the corresponding transgene. Importantly, this inventive combination of transgene delivery using a viral vector-based system and an EV-based system is exhibiting several surprising technical effects, notably the absence of immune responses triggered by the transgene when delivered using an EV-based vector, despite the transgene product triggering an immune response when delivered by the viral vector. Additionally, EV-based vectors which are endogenously loaded with RNA or DNA polynucleotides show an even lower tendency to trigger anti-transgene immune responses. This means that the present invention provides for a completely novel and unexpected solution to the problem of repeat, long-term gene therapy, i.e. employing EV-based delivery of a linear RNA or DNA molecule, e.g. mRNA or pDNA encoding for a POI (preferably loaded into the EV-based vector in an endogenous fashion), combined with AAV-based vtrab gene therapy delivery of the same transgene product, will enable long-term, safe and effective treatment without triggering humoral, innate or T cell-mediated immune responses against neither the viral vector nor the transgene.

Without wishing to be bound by any theory, it is surmised that the absence of any immune response against the transgene when delivered using EVs is a result of at least three mechanisms, namely (i) the absence of various viral genetic elements (such as, in the case of RNA viruses, folded RNA stabilizing domains) when delivering the transgene using an EV-based vector, (ii) non-TLR triggering internalization of EV-based vectors, and/or (iii) the presence of immuno-modulatory molecules, such as HLA-G, in EVs.

Thus, a preferred mode of administration of the transgene (and more specifically its transgene product) is initial delivery of the transgene using an AAV-based vector, followed by subsequent doses of the transgene delivered using the EV-based vector. Importantly, the inventors have also realized that one could apply the combination therapy to target different bodily compartments, for instance to administer the viral gene therapy to target the central nervous system (CNS) and the EV-based vector to target the periphery, or vice versa.

Without wishing to be bound by any particular illustration or example, the combinatorial treatments can be illustrated as follows:
- Parallel dosing of two pharmaceutical compositions: one composition comprising the viral vector comprising the transgene and another composition comprising the EV-based vector comprising the transgene in the form of a polynucleotide. The parallel dosing may be followed by one or more doses of the viral vector and advantageously by one of more doses of the EV-based vector.
- Sequential dosing of two pharmaceutical compositions: one composition comprising the viral vector comprising the transgene and in at least one subsequent administration another composition comprising the EV-based vector comprising the transgene in the form of a polynucleotide
- Parallel dosing of two pharmaceutical compositions: one composition comprising the viral vector comprising the transgene and another composition comprising the EV-based vector comprising the POI for which the transgene encodes, wherein the POI is present in the form of a polypeptide. The parallel dosing may be followed by one or more doses of the viral vector and advantageously by one of more doses of the EV-based vector, wherein the POI be present in the form of a protein or in the form of a polynucleotide corresponding to the transgene. Embodiment not part of the invention.
- Sequential dosing of two pharmaceutical compositions: one composition comprising the viral vector comprising the transgene and in at least one subsequent administration the EV-based vector comprising the POI for which the transgene encodes, wherein the POI is present in the form of a polypeptide. Embodiment not part of the invention.
- Parallel dosing of two pharmaceutical compositions: one composition comprising the viral vector comprising the transgene and another composition comprising the EV-based vector comprising the POI for which the transgene encodes, wherein the POI is present in the form of both a polypeptide and a transgene polynucleotide. The parallel dosing may be followed by one or more doses of the viral vector and advantageously by one of more doses of the EV-based vector, wherein the POI be present in the form of a protein and/or in the form of a polynucleotide corresponding to the transgene.
- Sequential dosing of two pharmaceutical compositions: one composition comprising the viral vector comprising the transgene and in at least one subsequent administration the EV-based vector comprising the POI for which the transgene encodes, wherein the POI is present in the form of a polypeptide and/or in the form of a polynucleotide transgene.

The present invention is highly suitable to be used in the treatment of paediatric genetic diseases, for instance non-limiting examples such as Niemann-Pick type C, Gaucher's disease types I, II, and III, urea cycle disorders such as argininosuccinic aciduria, and Duchenne's muscular dystrophy (DMD).

The references to methods of treatment in the following paragraphs are to be interpreted as references to the compounds and pharmaceutical compositions of the present invention for use in a method for treatment of the human (or animal) body by therapy.

In a further aspect, the present invention relates to a method of treatment comprising the steps of administering to a subject in need thereof (i) an AAV-based vector comprising the transgene (which encodes for the transgene product, i.e. the POI) and (ii) an EV-based vector comprising the transgene. Advantageously, as abovementioned, this invention is particularly suitable to treat genetic diseases, especially paediatric monogenetic diseases where a patient need to be treated more than once with the transgene in question. In particular, the present invention provides a highly safe and effective solution to the problem of patients having either pre-existing antibodies to a viral vector in question and/or when the patient develops antibodies to the viral vector after administration of said viral vector. A surprising but important aspect is that the present invention also solves the problem of patients developing antibodies to the transgene and/or to the POI expressed by the transgene, as this problem may have a detrimental impact on the safety and importantly the activity of the gene therapy.

In yet another aspect, the present invention relates to a method of manufacturing a combination therapy which comprises an AAV-based vector and an EV-based vector for delivery of the same transgene. The methods of the present invention typically comprise the steps of (a) infecting a first host cell with a viral vector genome comprising the transgene and collecting the viral vectors produced by said first host cell, and (b) genetically modifying a second host cell to produce EV-based vectors comprising said transgene in a DNA or RNA polynucleotide, for instance in an mRNA, a circRNA and/or pDNA polynucleotide, and collecting the EV-based vectors produced by the second host cell. The steps (a) and (b) may be carried out sequentially in any order, or in parallel, depending on what is the most cost and resource-effective approach. In one important embodiment, the host cells used in steps (a) and (b) may be the same cell type. Key to successfully avoiding an immune response against the transgene is the use of either endogenous EV-based vector loading strategies for the transgene, i.e. loading of the DNA or RNA polynucleotide such as mRNA, circRNA, mini-circle DNA and/or pDNA polynucleotide carrying the transgene through genetic engineering of the parental EV-producing cells, or highly efficient exogenous loading of the transgene in question, for instance in the form of mRNA, circRNA, mini-circle DNA, linear DNA polynucleotide and/or pDNA.

Thus, the present invention provides a completely unexpected solution to the problem of repeat administration of a transgene for gene therapy, with potentially considerable impact on the field of gene therapy.

### Brief description of the figures

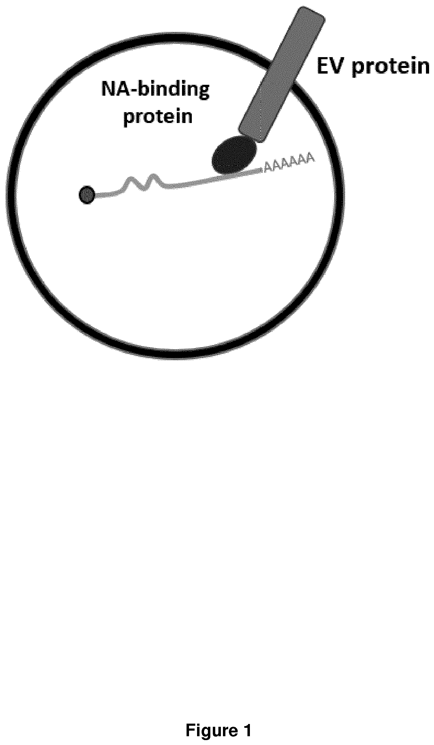

Figure 1 shows a schematic illustration of the loading of a transgene of interest into EV-based vectors, via endogenous genetic engineering of the EV-producing cell source. The illustration shows how a fusion protein between an EV protein and a nucleic acid-binding protein enables endogenous loading of in this case an mRNA transgene.
Figure 2 shows repeat delivery of a Nanoluciferase transgene using (in a first dose) an AAV vector and (in a second dose) an MSC-derived EV-based vector.
Figure 3 compares anti-transgene (i.e. anti-eGFP) antibody production against an eGFP transgene administered to C57BL/6 mice using either (a) two doses of AAVs or (b) one dose of AAV and one dose of an HEK293T-based EV endogenously loaded with an mRNA encoding for eGFP.

### Detailed description of the invention

The present invention is generally directed toward solving the problems associated with repeat administration of AAV-based viral gene therapy, in particular the need to overcome the production in patients of anti-vector and potentially even more importantly anti-transgene antibodies.

Thus, in a first aspect, the present invention relates to combination therapy, comprising
(i) an AAV-based viral vector comprising a transgene encoding for a protein of interest (POI), and
(ii) an EV-based vector comprising the transgene in the form of a DNA or RNA polynucleotide, for instance an mRNA, circRNA, mini-circle DNA, linear DNA, and/or pDNA. The transgene is initially administered using viral vector delivery followed by a second and optionally even further doses of the transgene using an EV-based vector. Importantly, the transgene may be delivered to different organs, tissues and/or bodily compartments using different delivery methods, i.e. using either viral vector delivery or delivery using an EV-based vector. As outlined in further detail below, the EV-based vector and the viral vector may be administered essentially simultaneously to the subject, as EVs often have immuno-modulatory effects which may be harnessed to dampen the immune response against the viral vector.

The transgenes of the present invention may normally encode for a protein, a polypeptide, or any combination thereof (a so called POI). In the case of viral gene therapy, delivery of a transgene, and the corresponding POI, often triggers the production of anti-POI antibodies, resulting in reduced activity and/or complete neutralization of the POI. The production of antibodies against either the transgene itself, the POI or the viral vector means that repeat dosing of viral gene therapies is conventionally not possible. However, the present invention provides an unexpected solution to this problem, through using EVs endogenously or exogenously loaded with a polynucleotide which comprises the same transgene, without triggering an immune response.

The terms "extracellular vesicle" or "EV" are used interchangeably herein and shall be understood to relate to any type of vesicle that is obtainable from a cell in any form, for instance a microvesicle (e.g. any vesicle shed from the plasma membrane of a cell), an exosome (e.g. any vesicle derived from the endosomal, lysosomal and/or endo-lysosomal pathway), an apoptotic body, ARMMs (arrestin domain containing protein 1 [ARRDC1]-mediated microvesicles), a microparticle and vesicular structures, etc. The EVs maybe genetically modified or genetically engineered, in this case the EVs are derived from a genetically modified/engineered producer cell usually comprising a recombinant transgene or protein product which is incorporated into the EVs produced by those cells. The term "modified" indicates that the vesicle has been modified either using genetic or chemical approaches, for instance via genetic engineering of the EV-producing cell or via e.g. chemical conjugation, for instance to attach moieties to the exosome surface. The sizes of EVs may vary considerably but an EV typically has a nano-sized hydrodynamic diameter, i.e. a diameter below 1000 nm. Clearly, EVs may be derived from any cell type, in vivo, ex vivo, and in vitro. Preferred EVs include exosomes and microvesicles, but other EVs may also be advantageous in various circumstances. Furthermore, said terms shall also be understood to relate to extracellular vesicle mimics, cell membrane-based vesicles obtained through for instance membrane extrusion, sonication, or other techniques, etc. Furthermore, when teachings herein refer to EVs in singular and/or to EVs as discrete natural nanoparticle-like vesicles it should be understood that all such teachings are equally relevant for and applicable to a plurality of EVs and populations of EVs.

In a preferred embodiment, the EV-based vector of the present invention is any extracellular vesicle (EV), for instance exosomes, microvesicles, or any combination thereof. A preferred embodiment of the present invention is to use a combinatorial therapy comprising an AAV vector for delivery of the transgene in question, combined with an EV-based vector delivery of the same transgene, which is sequentially.

In a highly preferred embodiment, the EV-based vectors of the combination therapy are endogenously modified to contain the transgene of interest. Preferably, the transgene is present in the form of an mRNA, a circRNA, a mini-circle DNA, and/or a pDNA, but other forms of DNA, RNA, or DNA/RNA polynucleotides may also be employed. Through genetic engineering of the EV-producing cell source, the transgene polynucleotide is endogenously loaded into the EVs as the vesicles are being produced by the cell, thereby allowing for non-immunogenic delivery of the transgene and the POI for which it encodes. The endogenous loading into the EVs is normally based on the use of a fusion protein between an EV protein and a nucleic acid (NA)-binding protein, wherein the NA-binding protein binds to (i.e. interacts with) and loads the RNA or DNA polynucleotide (preferably an mRNA, circRNA, mini-circle DNA, linear DNA and/or pDNA transgene) into the EVs. In preferred embodiments, the NA-binding protein comprised in the fusion protein is at least one of Cas6, Cas9, Cas13, PUF, PUF531, PUFx2, TALEN, a Zinc finger, a nuclease, a transcription factor, or any combination, domains, regions or mutants thereof. Importantly, exogenous loading into EVs of a polynucleotide encoding for a transgene is another tractable approach to transgene loading which appear to circumvent the problem of anti-transgene antibody production upon repeat administration of a transgene. Exogenous loading strategies for loading of mRNA, circRNA, mini-circle DNA, linear DNA and/or pDNA into EVs include electroporation, microfluidics, transfection into cells or directly into EVs already harvested from an EV-producing cell source, etc.

The combination therapy according to the present invention has considerable applicability for use in medicine, advantageously in the treatment of genetic and more specifically monogenetic diseases. Diseases where the combinatorial therapy of the present invention is particularly suitable include lysosomal storage disorders (LSDs), inherited errors of metabolism (IEMs), myopathies, mitochondrial diseases, peroxisomal diseases, inflammatory diseases, cancer, neurodegenerative diseases, neurological diseases, as well as essentially any other disease, ailment, condition, syndrome or disease state. Particular patient categories of relevance for the combination therapy of the present invention include patients that produce antibodies against the transgene, against cells into which the transgene is delivered, against the POI, or against the viral vector itself. Furthermore, patients in which innate, humoral, or T cell-mediated immune responses are triggered upon or after viral vector administration are highly suitable for follow-up treatment with the EV-based transgene delivery vectors of the present invention. Similarly, when a patient has known pre-existing antibodies to the viral vector or the transgene product the EV-based vectors per the present invention represent a highly tractable, non-immunogenic intervention. In addition, as a result of the different pharmacokinetics and pharmacodynamics profiles between the virus-based vectors and the EV-based vectors, different routes of administration and/or different formulations may be employed for the two components of the combination therapy (i.e. the viral and EV-based vectors). For instance, the viral vector may be administered into the CNS via intracerebroventricular or intrathecal administration whereas the EVs may be administered intravenously, subcutaneously, and/or intramuscularly, or vice versa depending on *inter alia* the type of viral vector, the type of EV-based vector, and the disease to be treated. As a non-limiting example, a suitable method of treatment of lysosomal storage disorders with neurological involvement may be to deliver the transgene encoding for the missing or defective enzyme or transporter (for instance GBA, NPC1, CLN6, IS2, LAMP2, alpha or beta mannosidase, etc.), using a viral vector administered into the CNS (for instance via intrathecal or intracerebroventricular administration) combined in parallel or sequentially with EV-based vector delivery of the RNA or DNA transgene in question, for instance administered systemically via i.v. or s.c. administration. Alternatively, as abovementioned, the viral and EV-based vectors may also be administered using the same route of administration, for instance intravenous administration, in separate pharmaceutical compositions. In a preferred embodiment, the viral vector-mediated delivery of the transgene is administered in a first dose, followed by the EV-based vector delivery of the corresponding transgene in a second and subsequent doses, wherein the second dose is given after the viral vector dose has been administered.

In a further aspect, the present invention relates to a method of treatment comprising the steps of administering to a subject in need thereof (i) an AAV-based vector comprising a transgene encoding for a POI and (ii) an EV-based vector comprising the transgene.

'Separate administration/delivery/dosing' can mean the administration of at least two active ingredients by different routes and at the same time or at substantially the same time.

'Sequential administration/delivery/dosing' can mean administration of at least two active ingredients at different times, the administration route being identical or different. More particularly, 'sequential' can mean the whole administration of one of the active ingredients is carried out before administration of the other or others commences. It is thus possible to administer one of the active ingredients over several months before administering the other active ingredient or ingredients. There is no simultaneous treatment in this case. An alternate administration of each active ingredient over several weeks can also be envisaged.

The terms "administration", "dosing", and "delivery" are used interchangeably in this specification. The combination therapy of the present invention may be administered to a human or animal subject via various different administration routes, for instance auricular (otic), buccal, conjunctival, cutaneous, dental, electro-osmosis, endocervical, endosinusial, endotracheal, enteral, epidural, extra-amniotic, extracorporeal, hemodialysis, infiltration, interstitial, intra-abdominal, intra-amniotic, intra-arterial, intra-articular, intrabiliary, intrabronchial, intrabursal, intracardiac, intracartilaginous, intracaudal, intracavernous, intracavitary, intracerebral, intracerebroventricular, intracisternal, intracorneal, intracoronal (dental), intracoronary, intracorporus cavernosum, intradermal, intradiscal, intraductal, intraduodenal, intradural, intraepidermal, intraesophageal, intragastric, intragingival, intraileal, intralesional, intraluminal, intralymphatic, intramedullary, intrameningeal, intramuscular, intraocular, intraovarian, intrapericardial, intraperitoneal, intrapleural, intraprostatic, intrapulmonary, intrasinal, intraspinal, intrasynovial, intratendinous, intratesticular, intrathecal, intrathoracic, intratubular, intratumor, intratym panic, intrauterine, intravascular, intravenous, intravenous bolus, intravenous drip, intraventricular, intravesical, intravitreal, iontophoresis, irrigation, laryngeal, nasal, nasogastric, occlusive dressing technique, ophthalmic, oral, oropharyngeal, other, parenteral, percutaneous, periarticular, peridural, perineural, periodontal, rectal, respiratory (inhalation), retrobulbar, soft tissue, subarachnoid, subconjunctival, subcutaneous, sublingual, submucosal, topical, transdermal, transmucosal, transplacental, transtracheal, transtympanic, ureteral, urethral, and/or vaginal administration, and/or any combination of the above administration routes, which typically depends on the disease to be treated and/or the characteristics of the pharmaceutical compositions to be administered.

As above-mentioned, this combinatorial approach is particularly relevant in patients who have pre-existing antibodies against the viral vector or the transgene product, in patients that develop antibodies against the viral vector or the transgene product, patients that suffer from a disease requiring reaching different organs, bodily compartments, or tissues with different vectors, etc. In one advantageous embodiment of the present invention, the viral vectors and the EV-based vectors may be administered via the same or via different routes of administration, in order to capitalize on the inherent advantages associated with the different delivery vectors.

In a further embodiment, the viral vector and the EV-based vector transgene delivery is carried out sequentially. The choice between administration regimens will depend on aspects such as actual or predicted antibody titres, general actual or predicted immunological response to the viral gene therapy, target organs to be reach by the different types of vectors, etc. In one preferred dosing regimen of the combination therapy, the viral vector-based delivery of the transgene is carried out 1 or at most 2 times or a handful of times, whereas the EV vector delivery of the transgene is carried out repeatedly, i.e. more than 1 time but normally more than 2 times or potentially for chronic, long-term treatment (i.e. administered tens to hundreds to thousands of times). The surprising realization that EV-based vectors endogenously loaded with a transgene in the form of an RNA or DNA polynucleotide (e.g. mRNA, circRNA, mini-circle DNA, linear DNA or pDNA) can be delivered without triggering an anti-transgene immune response means that viral gene therapy can be "topped up" using an EV-based vector approach over extended time periods, providing an optimal solution to the problem of repeat gene therapy administration.

In a further aspect, the present invention relates to a method of manufacturing the combination therapy of the present invention. The manufacturing methods of the present invention may typically comprise the steps of (a) infecting a first host cell with a viral vector genome comprising the transgene and collecting the viral vectors produced by said first host cell, and (b) genetically modifying a second host cell to produce EV-based vectors comprising said transgene in a DNA or RNA polynucleotide, for instance an mRNA, circRNA, mini-circle DNA, linear DNA and/or pDNA polynucleotide and collecting the EV-based vectors produced by the second host cell. The two steps may be carried out simultaneously, sequentially in any order, and/or in parallel.

In a preferred embodiment, the first host cell, which is used for AAV vector production, and the second host cell, which is used for EV-based vector production, are of the same cell type. Suitable cell sources include amnion-derived cells, placenta-derived cells, amniotic epithelial cells, mesenchymal stromal cells from e.g. bone marrow, adipose tissue, Wharton's jelly, HEK cells, etc. In a preferred embodiment, the genetic modification in step (b) comprises expressing in the second host cell (which may be of the same type as the first host cell, as abovementioned) a fusion protein comprising an EV protein and an NA-binding protein, wherein said NA-binding protein interacts with and loads the mRNA, other forms of linear RNA, circRNA, mini-circle DNA, linear DNA and/or pDNA transgene into the EV. In preferred embodiments, the NA-binding protein is selected from the group comprising at least one of Cas6, PUF, PUF531, PUFx2, Cas6, or any domain, segment, region, sequence, mutant, variant, or combination thereof. Generally, NA-binding proteins suitable in the context of the present invention include PUF, PUF531, PUFx2 (which comprises two PUF domains from human Pumilio 1 protein), PUFeng, DDX3X, EEF2, EEF1A1, HNRNPK, HNRNPM, HNRNPA2B1, HNRNHPH1, HNRNPD, HNRNPU, HNRNPUL1, NSUN2, Cas6, Cas13, Cas9, WDR1, HSPA8, HSP90AB1, MVP, PCB1, MOCS3, DARS, ELC2, EPRS, GNB2L1, IARS, NCL, RARS, RPL12, RPS18, RPS3, RUVBL1, TUFM, hnRNPA1, hnRNPA2B1, DDX4, ADAD1, DAZL, ELAVL4, ELAVL1, IGF2BP3, HNRNPQ, RBFOX1, RBFOX2, U1A, PPR family, ZRANB2, NUSA, IGF2BP1, IGF2BP2, Lin28, KSRP, SAMD4A, TDP43, FUS, FMR1, FXR1, FXR2, EIF4A1-3, MS2 coat protein, DEAD, KH, GTP_EFTU, dsrm, G-patch, IBN_N, SAP, TUDOR, RnaseA, MMR-HSR1, KOW, RnaseT, MIF4G, zf-RanBP, NTF2, PAZ, RBM1CTR, PAM2, Xpo1, Piwi, CSD, RipTALs , Bats and MOrTL1 and 2, BurrH, R2DBD, MBP1, Skn-1 bZL, ROR alpha 1, dnaA, and any combinations, derivatives, domains, regions, sites, mutated variants or part(s) thereof.

The EV proteins which is comprised in the fusion proteins as per the present invention may be selected from the group comprising the following non-limiting examples: CD9, CD53, CD63, CD81, CD54, CD50, FLOT1, FLOT2, CD49d, CD71, CD133, CD138, CD235a, ALIX, Syntenin-1, Syntenin-2, Lamp2b, TSPAN8, syndecan-1, syndecan-2, syndecan-3, syndecan-4, TSPAN14, CD37, CD82, CD151, CD231, CD102, NOTCH1, NOTCH2, NOTCH3, NOTCH4, DLL1, DLL4, JAG1, JAG2, CD49d/ITGA4, ITGB5, ITGB6, ITGB7, CD11a, CD11b, CD11c, CD18/ITGB2, CD41, CD49b, CD49c, CD49e, CD51, CD61, CD104, interleukin receptors, immunoglobulins, MHC-I or MHC-II components, CD2, CD3 epsilon, CD3 zeta, CD13, CD18, CD19, CD30, CD34, CD36, CD40, CD40L, CD44, CD45, CD45RA, CD47, CD86, CD110, CD111, CD115, CD117, CD125, CD135, CD184, CD200, CD279, CD273, CD 274, CD362, COL6A1, AGRN, EGFR, GAPDH, GLUR2, GLUR3, HLA-DM, HSPG2, L1CAM, LAMB1, LAMC1, ARRDC1, PDGFRN, ATP2B2, ATP2B3, ATP2B4, BSG, IGSF2, IGSF3, IGSF8, ITGB1, ATP1A2, ATP1A3, ATP1A4, ITGA4, SLC3A2, ATP1A1, ATP1B3, ATP2B1, LFA-1, LGALS3BP, Mac-1 alpha, Mac-1 beta, MFGE8, SLIT2, STX3, TCRA, TCRB, TCRD, TCRG, VTI1A, VTI1B, and any other EV proteins, and any combinations, derivatives, domains, variants, mutants, or regions thereof. Particularly advantageous EV proteins include CD63, CD81, CD9, CD82, CD44, CD47, CD55, LAMP2B, ICAMs, integrins, ARRDC1, syndecan, syntenin, and Alix, as well as derivatives, domains, variants, mutants, or regions thereof.

The host cells employed for the production of the viral vectors and the EV-based vectors may essentially be selected from any type of cell source, including primary cells, cell lines, cells present in a multicellular organism, or essentially any other type of cell source which can be utilized to produce viral vectors and/or EVs. The host cells may be grown in suspension culture, in adherent culture, on beads or other mobile elements, or any in other type of culturing system and in any type of vessel. Host cells as per the present invention may also include cells producing EVs and viral vectors in vivo. The host cells per the present invention may be selected from a wide range of cells, tissues and/or cell lines, for instance mesenchymal stromal cells (MSCs) (obtainable from e.g. bone marrow, adipose tissue, Wharton's jelly, perinatal tissue, placenta, tooth buds, umbilical cord blood, skin tissue, etc.), fibroblasts, amnion cells and more specifically amnion epithelial cells optionally expressing various early markers, myeloid suppressor cells, M2 polarized macrophages, adipocytes, endothelial cells, fibroblasts, human umbilical cord endothelial cells (HUVECs), human embryonic kidney (HEK) cells such as HEK293, HEK293T, HEK cells adapted to suspension culture and/or serum free culture, endothelial cell lines such as microvascular or lymphatic endothelial cells, erythrocytes, erythroid progenitors, chondrocytes, any cells obtained through amniocentesis or from the placenta, epithelial cells from the airways or alveolae, fibroblasts, endothelial cells, etc. Also, immune cells such as B cells, T cells, NK cells, macrophages, monocytes, dendritic cells (DCs) are also within the scope of the present invention, and essentially any type of cell which is capable of producing viral vectors and EVs is also encompassed herein. The host cells may be either allogeneic, autologous, or even xenogeneic in nature to the patient to be treated, i.e. the cells may be from the patient himself or from an unrelated, matched or unmatched donor.

In a further embodiment, the manufacturing method as per the present invention may additional comprise the step of formulating the AAV vector and/or the EV-based vector in a pharmaceutical composition. The two vector types may be formulated in the same type of pharmaceutical composition or may be formulated in different types of pharmaceutical compositions. Key to this choice is the route of administration, the dosages of the different vectors, and the disease to be treated. In one embodiment, the viral vector may be delivered at virtually the same time as the EV-based vector, in either the same pharmaceutical composition or in different pharmaceutical compositions. The formulation buffers of the present invention are typically aqueous solutions that contains one or more additives. Such additives may include a protein such as human serum albumin (HAS), a saccharide such as trehalose or sucrose, a high molecular weight structural additive (which may be a protein such as HSA), and a buffering component in water. As utilized within the context of the present invention, a "buffering compound" or "buffering component" should be understood to refer to a substance that functions to maintain the aqueous suspension at a desired pH. The aqueous solution may also contain one or more amino acids.

The formulation buffer may advantageously be used as a storage buffer for either one or both of the EV-based vector and the viral vector. Storage may take place at sub zero temperatures, such as -5 degrees Centigrade or cooler, preferably -20 degrees Centigrade or even more preferably -80 degrees Centigrade. The aqueous formulation of the EV-based vector and/or the viral vector in crude or purified form can alternatively be dried by lyophilization or evaporation at ambient temperature.

The solutions used for formulation, as previously described, may advantageously comprise one or more of a saccharide, a high molecular weight structural additive such as HSA, a buffering component, and water. The solution may also include one or more amino acids. The combination of these components acts to preserve the activity of the EV-based vectors and/or the viral vectors upon freezing, thawing, lyophilization, and/or administration and circulation. Although a preferred saccharide is trehalose, other saccharides may be used, such as lactose, sucrose, mannitol, glucose, inositol, fructose, maltose or galactose. In addition, combinations of saccharides can be used, for example, trehalose and mannitol, or sucrose and mannitol. The high molecular weight structural additive aids in preventing vector aggregation during freezing and provides structural support in the liquid, frozen or lyophilized state. Within the context of the present invention, structural additives are considered to be of "high molecular weight" if they are greater than 5000 Dalton. A preferred high molecular weight structural additive is human serum albumin. However, other substances may also be used, such as hydroxyethyl-cellulose, hydroxymethyl-cellulose, dextran, cellulose, gelatin, or povidone. Amino acids may function to further preserve vector infectivity upon cooling and thawing of the aqueous suspension. A preferred amino acid is arginine, but other amino acids such as lysine, omithine, serine, glycine, glutamine, asparagine, glutamic acid or aspartic acid can also be used. The buffering component acts to buffer the solution by maintaining a relatively constant pH. A variety of buffers may be used, depending on the pH range desired, preferably between 7.0 and 7.8. Suitable buffers include phosphate buffer and citrate buffer. In addition, it is preferable that the aqueous solution contain a neutral salt which is used to adjust the final formulation to an appropriate iso-osmotic salt concentration. Suitable neutral salts include sodium chloride, potassium chloride or magnesium chloride. A preferred salt is sodium chloride.

Downstream processing and purification of EV-based vectors may be achieved using methods selected from a group of techniques comprising liquid chromatography (LC), high-performance liquid chromatography (HPLC), bead-elute chromatography, spin filtration, diafiltration, tangential flow filtration (TFF), hollow fiber filtration, centrifugation, immunoprecipitation, flow field fractionation, dialysis, microfluidic-based separation, etc., or any combination thereof. In an advantageous embodiment, the purification of the EV-based vectors is carried out using a sequential combination of filtration (preferably diafiltration or ultrafiltration (UF) or tangential flow filtration (TFF)) and chromatography, for instance size exclusion LC, ion exchange LC and/or bead-elute LC. Combining purification steps normally enhances the purity of the resulting EV-based vector preparations and in turn leads to superior therapeutic activity.

Similarly, purification of viral vectors is well known in the art and include purification techniques such as centrifugation and chromatographic techniques. Other processes which are well known in the art can include concentration, filtration, diafiltration, or mixing with a suitable buffer or pharmaceutical excipient. Preparations of both the EV-based vectors and the viral vectors can be divided into unit dose and multi dose aliquots for distribution, which will retain the essential characteristics of the batch.The present invention may be advantageously utilized in numerous diseases, conditions and ailments, including but not limited to: alpha-mannosidosis, Aspartylglucosaminuria, Cholesteryl Ester Storage Disease, Cystinosis, Danon Disease, Fabry Disease, Farber Disease, Fucosidosis, Galactosialidosis, Gaucher Disease Type I, Gaucher Disease Type II, Gaucher Disease Type III, GM1 Gangliosidosis Type I, GM1 Gangliosidosis Type II, GM1 Gangliosidosis Type III, GM2 - Sandhoff disease, GM2 - Tay-Sachs disease, GM2 - Gangliosidosis, AB variant, Mucolipidosis II, Krabbe Disease, Lysosomal acid lipase deficiency, Metachromatic Leukodystrophy, MPS I - Hurler Syndrome, MPS I - Scheie Syndrome, MPS I Hurler-Scheie Syndrome, MPS II - Hunter Syndrome, MPS IIIA - Sanfilippo Syndrome Type A, MPS IIIB - Sanfilippo Syndrome Type B, MPS IIIB - Sanfilippo Syndrome Type C, MPS IIIB - Sanfilippo Syndrome Type D, MPS IV - Morquio Type A, MPS IV - Morquio Type B, MPS IX - Hyaluronidase Deficiency, MPS VI - Maroteaux-Lamy, MPS VII - Sly Syndrome, Mucolipidosis I - Sialidosis, Mucolipidosis IIIC, Mucolipidosis Type IV, Multiple Sulfatase Deficiency, Neuronal Ceroid Lipofuscinosis TI, Neuronal Ceroid Lipofuscinosis T2, Neuronal Ceroid Lipofuscinosis T3, Neuronal Ceroid Lipofuscinosis T4, Neuronal Ceroid Lipofuscinosis T5, Neuronal Ceroid Lipofuscinosis T6, Neuronal Ceroid Lipofuscinosis T7, Neuronal Ceroid Lipofuscinosis T8, Niemann-Pick Disease Type A, Niemann-Pick Disease Type B, Niemann-Pick Disease Type C, Pompe Disease, Pycnodysostosis, Salla Disease, Schindler Disease, Wolman Disease, Mitochondrial complex I deficiency, Mitochondrial complex II deficiency, Mitochondrial complex III deficiency, Mitochondrial complex IV deficiency, Mitochondrial complex V (ATP synthase) deficiency, Primary coenzyme Q10 deficiency (COQ10D), Cerebral, Ocular, Dental, Auricular, and Skeletal anomalies (CODAS) syndrome, Mitochondrial disease resulting from mutations in PoIG, Chronic Progressive External Ophthalmoplegia syndrome (CPEO), Alpers-Huttenlocher syndrome (AHS), Childhood Myocerebrohepatopathy Spectrum (MCHS), Myoclonic Epilepsy Myopathy Sensory Ataxia (MEMSA), Ataxia Neuropathy Spectrum (ANS), mitochondrial recessive ataxia syndrome (MIRAS), sensory ataxia neuropathy dysarthria and ophthalmoplegia (SANDO), Autosomal Recessive Progressive External Ophthalmoplegia (arPEO), Autosomal Dominant Progressive External Ophthalmoplegia (adPEO), Mitochondrial DNA depletion syndrome, Mitochondrial DNA depletion syndrome 1 MyoNeuro genie Gastrointestinal Encephalopathy (MNGIE), Mohr-Tranebjaerg syndrome, 3- methylglutaconic aciduria, Combined oxidative phosphorylation deficiency, Myopathy, Lactic Acidosis, and Sideroblastic Anemia (MLASA), Hyperuricemia, Pulmonary hypertension, Renal failure, and Alkalosis (HUPRA) syndrome, Leigh Syndrome, Leigh syndrome, French Canadian type, Friedreich ataxia, Gracile syndrome, Bjornstad syndrome, Multiple Mitochondrial Dysfunctions Syndrome (MMDS), Early-onset Ataxia with Ocular motor apraxia and Hypoalbuminemia (EAOH), Charcot-Marie-Tooth Disease-2A2, Leber Hereditary Optic Neuropathy (LHON), Sudden Infant Death Syndrome, Myoclonic Epilepsy with Ragged Red Fibers (MERRF), MERRF/MELAS overlap syndrome, Neuropathy, Ataxia, Retinitis Pigmentosa (NARP), Mitochondrial myopathy, Encephalomyopathy, Lactic Acidosis and Stroke-like episodes (MELAS), Leukoencephalopathy with Brain stem and Spinal cord involvement and Lactate elevation (LBSL), Mitochondrial disease resulting from SDH mutations, Spastic paraplegia, Paragangliomas, Pheochromocytoma, Optic atrophy type 1, Ethylmalonic encephalopathy, Carnitine-acylcarnitine translocase deficiency, Primary systemic carnitine deficiency, Cerebral creatine deficiency syndrome- 1, Cerebral creatine deficiency syndrome-2 or Cerebral creatine deficiency syndrome-3, Carnitine palmitoyltransferase 1 (CPT 1) deficiency, Carnitine palmitoyltransferase 2 (CPT II) deficiency, Short-chain acyl-CoA dehydrogenase deficiency, Very long chain acyl-CoA dehydrogenase deficiency, Long-chain 3-hydroxyl-CoA dehydrogenase (LCHAD) deficiency, Multiple acyl-CoA dehydrogenase deficiency, Glutaric acidemia IIA, Glutaric acidemia IIB, Glutaric acidemia IIC, Pyruvate carboxylase deficiency, Pymvate dehydrogenase deficiency, Pyruvate dehydrogenase EI -alpha deficiency, Pyruvate dehydrogenase phosphatase deficiency, Pyruvate dehydrogenase E3-binding protein deficiency, Pyruvate dehydrogenase E2 deficiency, Pyruvate dehydrogenase EI -beta deficiency, 3-hydroxyacyl-CoA dehydrogenase (SCHAD) deficiency/(HADH) deficiency, Cerebral creatine deficiency syndrome (CCDS) 1, CCDS 2, GLUT1 deficiency syndrome 1, infantile onset, severe, Epilepsy, progressive myoclonic 1A (Unverricht and Lundborg), Epilepsy, progressive myoclonic 2A (Lafora), Epilepsy, progressive myoclonic 2B (Lafora), Epileptic encephalopathy, early infantile (EIEE) Type 1, EIEE Type 3, EIEE Type 8, EIEE Type 12, EIEE Type 15, EIEE Type 16, EIEE Type 18, Multiple congenital anomalies-hypotonia-seizures syndrome 2 (MCAHS2) / EIEE Type 20, EIEE Type 21, EIEE Type 23, EIEE Type 25, EIEE Type 28, EIEE Type 29, EIEE Type 34, EIEE Type 35, Microcephaly, seizures, and developmental delay (MCSZ), Aicardi-Goutieres syndrome (AGS) Type 1, AGS Type 2, AGS Type 3, AGS Type 4, AGS Type 5, AGS Type 6, Congenital disorder of glycosylation (CDG), Type la, CDG Type Ib, CDG Type Ic, CDG Type Id, CDG Type le, CDG Type If, CDG Type Ig, CDG Type Ih, CDG Type Ii, CDG Type Ij, CDG Type Ik, CDG Type 11, CDG Type Im, CDG Type In, CDG Type Io, CDG Type Ip, CDG Type Iq, CDG Type Ir, CDG Type Is, CDG Type It, CDG Type lu, CDG Type Iw, CDG Type Ix, CDG Type ly, CDG Type Iz, CDG Type Ila, CDG Type lib, CDG Type lie, CDG Type lid, CDG Type lie, CDG Type Ilf, CDG Type Hg, CDG Type Ilh, CDG Type Iii, CDG Type Ilj, CDG Type Ilk, CDG Type III, CDG Type Iln, Spinocerebellar ataxia, autosomal recessive 1, Ataxia, early-onset, with oculomotor apraxia and hypoalbuminemia, Marinesco-Sjogren syndrome, Cockayne syndrome, type A / UV-sensitive syndrome 2, Cockayne syndrome, type B / Cerebrooculofacioskeletal syndrome 1 / De Sanctis-Cacchione syndrome / UV-sensitive syndrome, Spastic ataxia, Charlevoix-Saguenay type, Pelizaeus-Merzbacher disease / Spastic paraplegia 2, X-linked, Anemia, sideroblastic, with ataxia, Pontocerebellar hypoplasia (PCH), type la, PCH type Ib, PCH type Ic, PCH type 2a, PCH type 2b, PCH type 2c, PCH type 2d, PCH type 2e, PCH type 3, PCH type 4, PCH type 5, PCH type 6, PCH type 8, PCH type 9, PCH type 10, Spinocerebellar ataxia, autosomal recessive (SCAR) 2 (SCAR2), SCAR7, SCAR8, SCAR10, SCAR14, SCAR16, SCAR18, SCAR20, Ornithine transcarbamylase deficiency, Citrul!inemia, Argininosuccinic aciduria, Argininemia, Carbamoylphosphate synthetase I deficiency, Hemochromatosis, Menkes disease, Wilson disease, Molybdenum cofactor deficiency A, Molybdenum cofactor deficiency B, Neuro degeneration with brain iron accumulation (NBIA) 1 / HARP syndrome, NBIA 2B / Infantile neuroaxonal dystrophy 1 / Parkinson disease 14, autosomal recessive, NBIA 3, NBIA 4, NBIA 6, Kufor-Rakeb syndrome, Spastic paraplegia 35, Woodhouse-Sakati syndrome, Fucosidosis, Spastic paraplegia 30 / neuropathy, hereditary sensory type lie / mental retardation, autosomal dominant 9, Tyrosinemia (TYRSN), type 1, TYRSN type 2, TYRSN type 3, Homocysteinemia, Sulphite oxidase deficiency, Phenylketonuria, Maple syrup urine disease (MSUD), type la, MSUD, type Ib,- MSUD, type II, Glutaric aciduria, type I, Methylmalonic aciduria, mut(0) type, Holocarboxylase synthetase deficiency, Propionic academia, Isovaleric academia, Peroxisome biogenesis disorder 1A (Zellweger), Peroxisome biogenesis disorder 3A (Zellweger), Peroxisome biogenesis disorder 4A / 4B (Zellweger), Peroxisome biogenesis disorder 5A / 5B (Zellweger), Peroxisome biogenesis disorder 6A / 6B (Zellweger), Peroxisome biogenesis disorder 7A / 7B (Zellweger), Peroxisome biogenesis disorder 8 A / 8B (Zellweger), Peroxisome biogenesis disorder 10A (Zellweger), Peroxisome biogenesis disorder 11A / 11B (Zellweger), Peroxisome biogenesis disorder 12A (Zellweger), Peroxisome biogenesis disorder 13A (Zellweger), Adrenoleukodystrophy (ALD), Perrault syndrome (PRLT) 1, PRLTS1, PRLTS2, PRLTS3, PRLTS4, and PRLTS5.

The invention and its various aspects, embodiments, alternatives, and variants will now be further exemplified and illustrated with the enclosed non-limiting examples, which naturally also may be modified considerably without departing from the scope and the gist of the invention.

### Experimental section and examples

### Vector construct design and cloning

As a non-limiting example merely for illustrative purposes, for loading and delivery of a transgene in mRNA format using EV-based vectors, various NA-binding domains and variants thereof (e.g. PUF, PUFeng (an engineered version of a PUF protein), Cas6, Cas13, etc.) were used in combination with several exosomal polypeptides (such as CD81, CD63, CD9, syntenin, syndecan, Alix, CD133, etc.). ORFs were typically generated by synthesis and cloned into the mammalian expression vector pSF-CAG-Amp. Briefly, synthesized DNA and vector plasmid were digested with enzymes Notl and Sall as per manufacturers instruction (NEB). Restricted, purified DNA fragments were ligated together using T4 ligase as per manufacturers instruction (NEB). Successful ligation events were selected for by bacterial transformation on ampicillin-supplemented plates. Plasmid for transfection was generated by 'maxi-prep', as per manufacturers instruction.

Viral vectors were typically obtained from commercial manufacturers of viral vectors, for instance Addgene, Oxford Genetics, or Vector Biolabs. Briefly, as illustrated below for AAV vectors, the gene of interest (for instance reporters such as NanoLuciferase or GFP, or GOIs encoding for therapeutic POIs such as NPC1 or GBA) was cloned into a pAAV cis plasmid. The pAAV cis plasmid and helper plasmids (Ad helper vector and AAV rep/cap vector) were subsequently prepared at larger scale using a mega or maxi prep approach, followed by transfection into suitable virus-producing cells, such as HEK293T cells, in 24-well plates. AAV serotypes of interest include serotypes 1, 2, 3, 4, 5, 6, 7, 8, 9, 10 and synthetic AAV serotypes such as AAV-DJ or AAV6.2, etc. Other viral vectors of interest beyond AAVs include lentiviral vectors, adenoviral vectors, retroviruses, as well as various other viral vectors.

### Cell culture and transfection

Depending on the experimental design and assays, cell culture included transducing cells to stably express EV-based vectors or viral vectors, wherein in other cases transient transfection and EV production was sufficient. The producer cells for the viral and EV-based vectors were primarily grown in suspension culture or in adherent culture, on beads or other mobile elements, or any in other type of culturing system and in any type of vessel. The producing cells per the present invention may be selected from a wide range of cells, tissues and/or cell lines, for instance mesenchymal stromal cells (MSCs) (obtainable from e.g. bone marrow, adipose tissue, Wharton's jelly, perinatal tissue, placenta, tooth buds, umbilical cord blood, skin tissue, etc.), fibroblasts, amnion cells and more specifically amnion epithelial cells optionally expressing various early markers, myeloid suppressor cells, M2 polarized macrophages, adipocytes, endothelial cells, fibroblasts, human umbilical cord endothelial cells (HUVECs), human embryonic kidney (HEK) cells such as HEK293, HEK293T, HEK cells adapted to suspension culture and/or serum free culture, endothelial cell lines such as microvascular or lymphatic endothelial cells, erythrocytes, erythroid progenitors, chondrocytes, any cells obtained through amniocentesis or from the placenta, epithelial cells from the airways or alveolae, fibroblasts, endothelial cells, etc. For conciseness, only a few examples are mentioned herein. HEK293T cells were typically seeded into 15 cm dishes (9×10⁶ cells per dish) and left overnight in serum-containing DMEM as recommended by ATCC. The following day the cells were transiently transfected with lipoplexed DNA added directly onto cells. Briefly, DNA and polyethyleneimine (PEI) were separately incubated in OptiMEM for 5 minutes before combining together for 20 minutes at room temperature. Lipoplexed DNA and cells were co-incubated for 6 hours following which conditioned culture media was changed to OptiMEM for 48 hours. Other cells and cell lines that were evaluated in dishes, flasks and other cell culture vessels included bone marrow-derived mesenchymal stromal cells (BM-MSCs) and Wharton's jelly-derived MSCs (WJ-MSCs), amnion cells, amnion epithelial cells, fibroblasts, various endothelial and epithelial cells, as well as various immune cells and cell lines.

In the case of viral transduction and creation of stable cell lines for creating EV-based vector production systems, cell sources such as BM-MSCs, WJ-MSC, fibroblasts, amnion epithelial cells, fibroblasts, various endothelial and epithelial cells, were virus-transduced, typically using lentivirus (LV) transduction. Typically, 24 hours before infection, 100.000 cells (e.g. fibroblasts, MSCs, etc.) or 200.000 cells (e.g. HEK293T) are plated in a 6-well plate. 2 uL of LV and optionally Polybrene (or hexadimethrine bromide, final concentration on the well of 8 ug/mL) are added, and 24 hours post transduction the cell medium of transduced cells is changed to fresh complete media. At 72 hours post transduction, puromycin selection (4-6pg/ml) is performed, normally for 7 days followed by analysis of stable expression of the fusion polypeptides. Stable cells were also created using non-viral transfection methods, including electroporation, lipofection, transfection with polycationic agents such as PEI, etc. Stably transfected cells were typically cultured in either 2D culture, in suspension bioreactors or in adherent cell bioreactors and conditioned media was subsequently harvested for EV-based vector preparation. Various preparation and purification steps were carried out as above-mentioned, with the standard workflow comprising the steps of pre-clearing of the supernatant, filtration-based concentration, chromatography-based removal of contaminants, and optional formulation of the resultant exosome composition in a suitable buffer for *in vitro* and/or *in vivo* assays.

### Assays and analytics

Western blot, qPCR and RNA seq are powerful and convenient analytical methods to evaluate the enrichment of transgenes in EVs. Briefly, for Western blot, SDS-PAGE was performed according to manufacturer's instruction (Invitrogen, Novex PAGE 4-12% gels), whereby 1×10¹⁰ EVs and 20 ug cell lysate were loaded per well. Proteins from the SDS-PAGE gel were transferred to PVDF membrane according to manufacturer's instruction (Immobilon (RTM), Invitrogen). Membranes were blocked in Odyssey blocking buffer (Licor) and probed with antibodies against NA-binding domain polypeptide (for instance PUF) and/or the exosomal protein according to supplier's instruction (Primary antibodies - Abcam, Secondary antibodies - Licor). Molecular probes visualized at 680 and 800 nm wavelengths. qPCR was carried out using RNA extracted from vectors or target cells. Reverse transcription was performed using an oligodT primer for mRNA. For NanoLuc quantification in Example 1, an amplicon corresponding to a region in the 5' end of the Nanoluc mRNA was then quantified using qPCR and absolute quantification was carried out against a standard curve.

For EV size determination, nanoparticle tracking analysis (NTA) was performed with a NanoSight (RTM) instrument equipped with analytical software, or occasionally on with a Particle Metrics machine. For recordings on the NanoSight (RTM), a camera level of 13 or 15 and automatic function for all post-acquisition settings were used. Electron microscopy and fluorescence microscopy were frequently used to validate and assess EV or virus morphology and size.

As abovementioned, EVs were isolated and purified using a variety of methods, typically a combination of filtration such as TFF and size exclusion liquid chromatography, ionic exchange chromatography and/or bead-elute liquid chromatography. Typically, EV-containing media was collected and subjected to a low speed spin at 300g for 5 minutes, followed by 2000g spin for 10 minutes to remove larger particles and cell debris. The supernatant was then filtered with a 0.22 µm syringe filter and subjected to different purification steps. Large volumes were diafiltrated and concentrated to roughly 20 ml using the Vivaflow 50R tangential flow (TFF) device (Sartorius) with 100 kDa cutoff filters or the KR2i TFF system (SpectrumLabs) with 100 or 300 kDa cutoff hollow fibre filters. The preconcentrated medium was subsequently loaded onto the bead-eluate columns (HiScreen (RTM) or HiTrap (RTM) Capto Core 700 column, GE Healthcare Life Sciences), connected to an ÄKTAprime (RTM) plus or ÄKTA Pure 25 (RTM) chromatography system (GE Healthcare Life Sciences). Flow rate settings for column equilibration, sample loading and column cleaning in place procedure were chosen according to the manufacturer's instructions. The sample was collected according to the UV absorbance chromatogram and concentrated using an Amicon Ultra-15 10 kDa molecular weight cut-off spin-filter (Millipore) to a final volume of 100 µl and stored at -80°C for further downstream analysis. To assess the protein and RNA elution profiles, media was concentrated and diafiltrated with KR2i TFF system using 100 kDa and 300 kDa hollow fibre filters and a sample analysed on a Tricorn 10/300 Sepharose 4 Fast Flow (S4FF) column (GE Healthcare Life Sciences).

### Example 1: Repeat delivery of a Nanoluciferase transgene using AAVs and MSC EVs

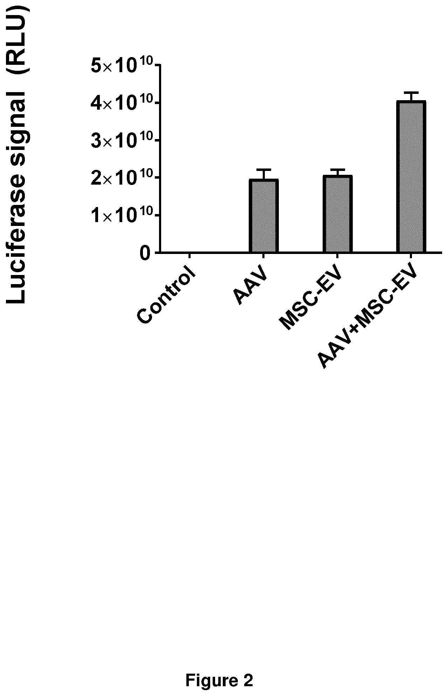

AAV vectors were prepared by using an adenoviral Nanoluciferase expression construct in HEK293T producer cells according to standard protocols. Human immortalised MSCs were engineered by lentiviral gene transfer to stably express a fusion construct between CD63 and the NA-binding proteins Cas13 or PUF, resulting in endogenous packaging of nanoluciferase mRNA into EVs. EVs were isolated and concentrated with tangential flow filtration (TFF) and bead-elute chromatography protocols. Both AAV vectors and isolated EVs were subjected to nanoparticle tracking analysis to determine particle concentrations. 1e10 AAV particles, or 1e10 EVs, or both 1e10 AAV particles and 1e10 EVs were then added together with fresh culture medium to 6 wells seeded with Huh7 cells at a confluency of 50%. Cells were incubated for 36 hours at 37°C and 5% CO₂, afterwards washed two times with PBS. Cells were lysed, substrate was added and luciferase signals were quantified according to the manufacturer's instructions (Promega). Results are shown in Figure 2, indicating successful and comparable delivery and translation of nanoluciferase mRNA to Huh7 cells when using either AAV or MSC-EVs, or both. Control values correspond to Huh7 cells without AAV/EVs added. When 1e10 AAVs or 1e10 MSC-EVs are added, the amount of expressed transgene in Huh7 cells was comparable. Treatment (n=3) with EVs in addition to AAV resulted in almost 2x transgene expression, i.e. luciferase signals.

### Example 2: Anti-eGFP antibody production differs between AAV and EV-mediated gene therapy

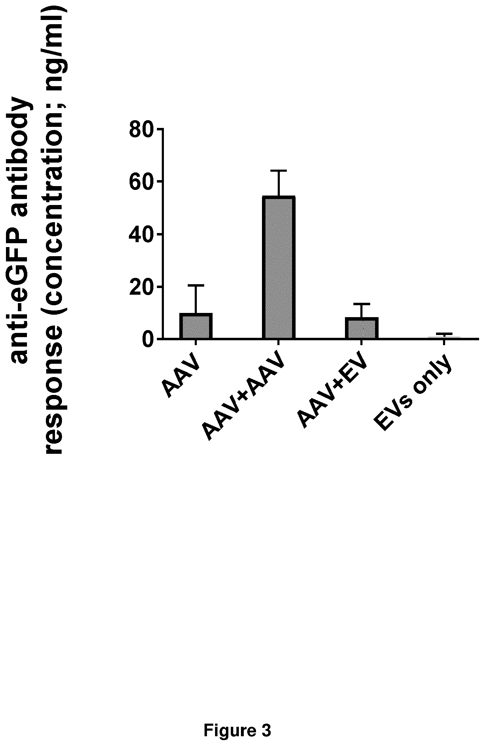

This example set out to verify that combinatorial gene therapy using a combination of a viral vector (in this case AAV9) for transgene delivery and an EV-based vector for delivery of the same transgene enables repeat administration in vivo, without anti-transgene antibodies being raised against the second dose. EV-mediated transgene delivery following AAV9-mediated delivery was compared to re-adminstration of the AAV vector, with the AAV vector encoding for eGFP and the EV-based vector endogenously loaded with the corresponding mRNA encoding for eGFP (using a CD81-Cas6 fusion protein for mRNA loading) were prepared by the same principle as above, but here both vector types were produced in HEK293T host cells. An in vitro antibody ELISA assay for quantification of anti-transgene antibody production against eGFP was performed on blood samples taken from C57BL/6 mice after (i) administration of the AAV9eGFP vector (1E10 AAV9 particles) once (AAV in Figure 3), (ii) after repeat administration of the AAV9eGFP vector (AAV+AAV in Figure 3), and (iii) after sequential administration of AAV9eGFP and HEK-EV-eGFPmRNA (1E10 EV particles) (AAV+EV). Serial dilutions with standards were done to create a standard curve that allowed to measure the antibody concentration released by PBMCs. As can be seen from Figure 3, repeat administration of the AAV9eGFP vectors resulted in a considerable induction in anti-eGFP antibody production, whereas the combination therapy using the AAV9eGFP vector and the HEK-EV-eGFPmRNA showed essentially no additional anti-transgene antibody production as compared to AAV9eGFP alone.

## Claims

1. A combination therapy, comprising (i) a viral vector comprising a transgene encoding for a protein of interest, and (ii) an extracellular vesicle (EV)-based vector comprising the transgene in the form of mRNA, circular RNA (circRNA), mini-circle, plasmid DNA (pDNA) or a linear DNA polynucleotide for use in medicine, wherein the EV-based vector is produced from a cell source, such as a cell line, that is allogenic in nature to the patient to be treated, and wherein the transgene is initially administered using the viral vector and subsequently administered using the EV- based vector; further wherein the viral vector is an adeno-associated virus (AVV)-based vector.

2. A combination therapy, comprising (i) a viral vector comprising a transgene encoding for a protein of interest, and (ii) an extracellular vesicle (EV)-based vector comprising the transgene in the form of mRNA, circular RNA (circRNA), mini-circle, plasmid DNA (pDNA) or a linear DNA polynucleotide for use in the treatment of a genetic disease, wherein the EV-based vector is produced from a cell source, such as a cell-line, that is allogenic in nature to the patient to be treated, and wherein the transgene is initially administered using the viral vector and subsequently administered using the EV- based vector; further wherein the viral vector is an adeno-associated virus (AVV)-based vector.

3. A combination therapy for use, according to claim 2, wherein the genetic disease is selected from the group consisting of lysosomal storage disorders, inherited errors of metabolism, myopathies, peroxisomal disorders, mitochondrial diseases, neurological and neurodegenerative diseases, inflammatory diseases and cancer.

4. A combination therapy for use, according to any one of the preceding claims, wherein the transgene encodes for a peptide or a polypeptide.

5. A combination therapy for use, according to any one of the preceding claims, wherein the EV-based vector is, an exosome, or a microvesicle.

6. A combination therapy for use, according to any one of the preceding claims, wherein the EV-based vector is exogenously or endogenously modified to contain the transgene.

7. A combination therapy for use, according to claim 6, wherein the EV-based vector is modified to comprise a fusion protein comprising an EV protein and a nucleic acid (NA)- binding protein, wherein said NA-binding protein interacts with and loads the mRNA, circRNA, mini-circle, pDNA or linear DNA polynucleotide transgene into the EV-based vector.

8. A method of manufacturing a combination therapy, according to any one of claims 1- 7, comprising the steps of:
(a) infecting a first host cell with a viral vector genome comprising the transgene and collecting the viral vectors produced by said first host cell; and,
(b) genetically modifying a second host cell, wherein the second host cell is allogenic in nature to the patient to be treated, to produce EV-based vectors comprising said transgene in an mRNA, circRNA, mini-circle, pDNA or linear DNA polynucleotide and collecting the EV-based vectors produced by the second host cell,
wherein steps (a) and (b) may be carried out sequentially in any order or in parallel.

9. The method of claim 8, wherein the first host cell and the second host cell are of the same cell type.

10. The method according to claim 8 or claim 9, wherein the genetic modification in step (b) comprises expressing in the second host cell a fusion protein comprising an EV protein and a NA-binding protein, wherein said NA-binding protein interacts with and loads the mRNA, circRNA, mini-circle DNA, pDNA or linear DNA polynucleotide transgene into the EV-based vector.

11. The method according to any one of claims 8-10, further comprising the step of formulating the viral vector and the EV-based vector in one or more pharmaceutical composition.

## Patentansprüche

1. Kombinationstherapie, umfassend (i) einen viralen Vektor, der ein Transgen umfasst, das für ein Protein von Interesse codiert, und (ii) einen Vektor auf der Basis eines extrazellulären Vesikels (EV), der das Transgen in Form von mRNA, zirkulärer RNA (circRNA), Minicircle, Plasmid-DNA (pDNA) oder einem linearen DNA-Polynukleotid umfasst, zur Verwendung in der Medizin, wobei der Vektor auf EV-Basis aus einer Zellquelle, wie z. B. einer Zelllinie, hergestellt wird, die für den zu behandelnden Patienten allogener Natur ist, und wobei das Transgen zunächst unter Verwendung des viralen Vektors und anschließend unter Verwendung des Vektors auf EV-Basis verabreicht wird; wobei der virale Vektor zudem ein Vektor auf der Basis eines adenoassoziierten Virus (AVV) ist.

2. Kombinationstherapie, umfassend (i) einen viralen Vektor, der ein Transgen umfasst, das für ein Protein von Interesse codiert, und (ii) einen Vektor auf der Basis eines extrazellulären Vesikels (EV), der das Transgen in Form von mRNA, zirkulärer RNA (circRNA), Minizirkel, Plasmid-DNA (pDNA) oder einem linearen DNA-Polynukleotid umfasst, zur Verwendung bei der Behandlung einer genetischen Krankheit, wobei der Vektor auf EV-Basis aus einer Zellquelle, wie z. B. einer Zelllinie, hergestellt wird, die für den zu behandelnden Patienten allogener Natur ist, und wobei das Transgen zunächst unter Verwendung des viralen Vektors und anschließend unter Verwendung des Vektors auf EV-Basis verabreicht wird; wobei der virale Vektor zudem ein Vektor auf Basis eines adenoassoziierten Virus (AVV) ist.

3. Kombinationstherapie zur Verwendung nach Anspruch 2, wobei die genetische Krankheit ausgewählt ist aus der Gruppe bestehend aus lysosomalen Speicherstörungen, vererbten Stoffwechselstörungen, Myopathien, peroxisomalen Störungen, mitochondrialen Krankheiten, neurologischen und neurodegenerativen Krankheiten, entzündlichen Krankheiten und Krebs.

4. Kombinationstherapie zur Verwendung nach einem der vorhergehenden Ansprüche, wobei das Transgen für ein Peptid oder ein Polypeptid codiert.

5. Kombinationstherapie zur Verwendung nach einem der vorhergehenden Ansprüche, wobei der Vektor auf EV-Basis ein Exosom oder ein Mikrovesikel ist.

6. Kombinationstherapie zur Verwendung nach einem der vorhergehenden Ansprüche, wobei der Vektor auf EV-Basis derart exogen oder endogen modifiziert ist, dass er das Transgen enthält.

7. Kombinationstherapie zur Verwendung nach Anspruch 6, wobei der Vektor auf EV-Basis derart modifiziert ist, dass er ein Fusionsprotein umfasst, das ein EV-Protein und ein Nukleinsäure-(NA)-bindendes Protein umfasst, wobei das NA-bindende Protein mit dem mRNA-, circRNA-, pDNA- oder dem linearen DNA-Polynukleotid-Transgen interagiert und diese in den Vektor auf EV-Basis lädt.

8. Verfahren zur Herstellung einer Kombinationstherapie nach einem der Ansprüche 1 bis 7, umfassend die Schritte:
(a) Infizieren einer ersten Wirtszelle mit einem viralen Vektorgenom, das das Transgen enthält, und Sammeln der von der ersten Wirtszelle produzierten viralen Vektoren; und
(b) genetisches Modifizieren einer zweiten Wirtszelle, wobei die zweite Wirtszelle von Natur aus für den zu behandelnden Patienten allogener Natur ist, so dass Vektoren auf EV-Basis produziert werden, die das Transgen in einem mRNA-, circRNA-, Minicircle-, pDNA- oder linearen DNA-Polynukleotid umfassen, und Sammeln der von der zweiten Wirtszelle produzierten Vektoren auf EV-Basis,
wobei die Schritte (a) und (b) nacheinander in beliebiger Reihenfolge oder parallel durchgeführt werden können.

9. Verfahren nach Anspruch 8, wobei die erste Wirtszelle und die zweite Wirtszelle vom gleichen Zelltyp sind.

10. Verfahren nach Anspruch 8 oder 9, wobei die genetische Modifikation in Schritt (b) die Expression eines Fusionsproteins in der zweiten Wirtszelle umfasst, das ein EV-Protein und ein NA-bindendes Protein umfasst, wobei das NA-bindende Protein mit dem mRNA-, circRNA-, Minicircle-DNA-, pDNA- oder linearen DNA-Polynukleotid-Transgen interagiert und diese in den Vektor auf EV-Basis lädt.

11. Verfahren nach einem der Ansprüche 8 bis 10, zudem umfassend den Schritt der Formulierung des viralen Vektors und des Vektors auf EV-Basis in einer oder mehreren pharmazeutischen Zusammensetzungen.

## Revendications

1. Thérapie combinée, comprenant (i) un vecteur viral comprenant un transgène codant pour une protéine d'intérêt, et (ii) un vecteur à base de vésicules extracellulaires (EV) comprenant le transgène sous la forme d'ARNm, d'ARN circulaire (ARNcirc), de mini-cercle, d'ADN de plasmide (ADNp) ou d'un polynucléotide d'ADN linéaire pour une utilisation en médecine, le vecteur à base d'EV étant produit à partir d'une source de cellules, telle qu'une lignée cellulaire, qui est de nature allogénique par rapport au patient à traiter, et le transgène étant initialement administré en utilisant le vecteur viral et subséquemment administré en utilisant le vecteur à base d'EV ; en outre, le vecteur viral étant un vecteur à base de virus adéno-associé (AVV).

2. Thérapie combinée, comprenant (i) un vecteur viral comprenant un transgène codant pour une protéine d'intérêt, et (ii) un vecteur à base de vésicules extracellulaires (EV) comprenant le transgène sous la forme d'ARNm, d'ARN circulaire (ARNcirc), de mini-cercle, d'ADN de plasmide (ADNp) ou d'un polynucléotide d'ADN linéaire pour une utilisation dans le traitement d'une maladie génétique, le vecteur à base d'EV étant produit à partir d'une source de cellules, telle qu'une lignée cellulaire, qui est de nature allogénique par rapport au patient à traiter, et le transgène étant initialement administré en utilisant le vecteur viral et subséquemment administré en utilisant le vecteur à base d'EV ; en outre, le vecteur viral étant un vecteur à base de virus adéno-associé (AVV).

3. Thérapie combinée pour une utilisation selon la revendication 2, la maladie génétique étant choisie dans le groupe constitué par des troubles du stockage lysosomal, des erreurs héritées du métabolisme, des myopathies, des troubles du peroxysome, des maladies mitochondriales, des maladies neurologiques et neurodégénératives, des maladies inflammatoires et un cancer.

4. Thérapie combinée pour une utilisation selon l'une quelconque des revendications précédentes, le transgène codant pour un peptide ou un polypeptide.

5. Thérapie combinée pour une utilisation selon l'une quelconque des revendications précédentes, le vecteur à base d'EV étant un exosome ou une microvésicule.

6. Thérapie combinée pour une utilisation selon l'une quelconque des revendications précédentes, le vecteur à base d'EV étant modifié de manière exogène ou de manière endogène pour contenir le transgène.

7. Thérapie combinée pour une utilisation selon la revendication 6, le vecteur à base d'EV étant modifié pour comprendre une protéine de fusion comprenant une protéine EV et une protéine de liaison à un acide nucléique (NA), ladite protéine de liaison à un NA interagissant avec et chargeant le transgène d'ARNm, d'ARNcirc, de mini-cercle, d'ADNp ou d'un polynucléotide d'ADN linéaire dans le vecteur à base d'EV.

8. Procédé de fabrication d'une thérapie combinée selon l'une quelconque des revendications 1 à 7, comprenant les étapes de :
(a) infection d'une première cellule hôte avec un génome de vecteur viral comprenant le transgène et collecte des vecteurs viraux produits par ladite première cellule hôte ; et,
(b) modification de manière génétique d'une deuxième cellule hôte, la deuxième cellule hôte étant de nature allogénique par rapport au patient à traiter, pour produire des vecteurs à base d'EV comprenant ledit transgène dans un ARNm, un ARNcirc, un mini-cercle, un ADNp ou un polynucléotide d'ADN linéaire et collecte des vecteurs à base d'EV produits par la deuxième cellule hôte,
les étapes (a) et (b) pouvant être réalisées séquentiellement selon un ordre quelconque ou en parallèle.

9. Procédé selon la revendication 8, la première cellule hôte et la deuxième cellule hôte étant du même type de cellules.

10. Procédé selon la revendication 8 ou la revendication 9, la modification génétique dans l'étape (b) comprenant l'expression dans la deuxième cellule hôte d'une protéine de fusion comprenant une protéine EV et une protéine de liaison à un NA, ladite protéine de liaison à un NA interagissant avec et chargeant le transgène d'ARNm, d'ARNcirc, d'ADN de mini-cercle, d'ADNp ou d'un polynucléotide d'ADN linéaire dans le vecteur à base d'EV.

11. Procédé selon l'une quelconque des revendications 8 à 10, comprenant en outre l'étape de formulation du vecteur viral et du vecteur à base d'EV dans une ou plusieurs compositions pharmaceutiques.
